# EUROPEAN PATENT APPLICATION

(11) **EP 0 759 471 A1**
(43) Date of publication of application: **26.02.1997**
(21) Application number: 95918162.9
(22) Date of filing: 10.05.1995
(51) Int. Cl.: C12N 15/49, C12N 15/86

(54) **RECOMBINANT HUMAN IMMUNODEFICIENCY VIRUS VECTOR AND PROCESS FOR PRODUCING THE SAME**

(30) Priority: 10.05.1994 JP 96794/94
(71) Applicant: HISAMITSU PHARMACEUTICAL CO. INC., Tosu-shi Saga 841 (JP)
(72) Inventor: SHIMADA, Takashi Nippon Medical School, Tokyo 113 (JP); AKIYAMA, Katsuhiko Tsukuba Institute of Hisamitsu, Tsukuba-shi Ibaragi 305 (JP)
(74) Representative: Pett, Christopher Phineas
(86) International application number: JP9500893
(87) International publication number: WO9530755

(57) **Abstract**

A recombinant HIV vector which retains the foreign DNA sequence antagonistic against HIV growth and/or gene expression and wherein the U3 domain of the LTR sequence on the genome 5' side has been replaced by the promoter sequence of a cytomegalovirus. As this vector can express an anti-HIV sequence of, for example, antisense, ribozyme or TAR decoy in HIV-infected cells without fail, it is useful as a material for gene therapy of HIV infection.

## Description

### TECHNICAL FIELD

The present invention relates to a recombinant human immunodeficiency viral vector and a manufacturing method thereof. More particularly, the present invention relates to a novel human immunodeficiency virus (hereinafter abbreviated as "HIV") vector which is useful for genetic therapy against infectious diseases of HIV and a method of manufacturing same.

### BACKGROUND ART

Along with the recent progress made in gene introducing technology into cells, trials to apply such technology to genetic therapy are being actively made. Genetic therapy is a technique of applying therapy against a disease by introducing in vivo a recombinant gene antagonistically acting on causative gene of the disease for expression. In the United States, genetic therapy has already been started against adenosine deaminase (ADA) deficiency disease, low-density lipoprotein receptor deficiency disease, such hereditary diseases as cystic fibrosis, and cancers such as brain tumor and malignant melanoma. More recently, furthermore, many fundamental studies are made of genetic therapy against virus infectious diseases including AIDS.

Among the currently applied gene introducing techniques in these genetic therapeutic treatments, a method using Molony Murine Leukemia Virus (hereinafter abbreviated as "MoMLV"), which is a retrovirus of mouse, as the vector forms the main current. An advantage of using this MoMLV vector is the possibility to introduce genes into many kinds of cells, which permits wide application to various diseases to be covered by genetic therapy. In the case of gene introduction in vivo such as intravenous or intramuscular administration, however, the essential nature of this vector of not having tissue-specificity becomes in contrast a defect: even by administering this MoMLV vector directly to a patient, the vector is trapped by cells near the administered portion, so that it becomes difficult to introduce the gene into the target cell. Further, introduction of a recombinant gene into a cell other than the target one may cause a side effect. For these reasons, it is the usual practice, as a basic protocol of the current genetic therapy using MoMLV vector, to use autogenous transplantation, i.e., to take out the target cell ex vivo from the patient, culture it, introduce the vector in vitro, and subsequently introduce the cell back into the patient. This practice however still involves such problems in that: (1) depending upon the kind of cell, it may be difficult to take out the cell ex vivo or it takes much time and labor to select only the target cell, and that (2) a special equipment for a germfree culture is required and facilities in which a therapy is applicable are limited.

As a method for solving these problems, the use of a tissue-specific virus vector is now studied. More specifically, HIV, which is a human retrovirus, causes infection through combination of gp120 which is a virus envelope protein with CD4 molecule present on the surface of a human CD4 positive T cell. There is therefore proposed a new recombinant virus vector called HIV vector utilizing this specific infection mechanism (Shimada, T., et al., J. Clin. Invest., 88, 1043, 1991). The most important feature of HIV vector is that it permits introduction of an exotic gene specifically at a high efficiency into CD4 positive T cell. Since it is possible to introduce in vivo this tissue-specific vector into a target cell by administering directly to a patient, no special equipment is required, thus making it possible to apply medical actions to an ordinary outpatient. This vector is therefore expected to make a remarkable contribution to genetic therapy hereafter with a CD4 positive T cell as the target cell.

Diseases for which therapeutic effect is expected from genetic therapy include all those becoming apparent, whether congenital or acquired, by a causative abnormality of genes. It may be considered a therapeutic method very useful particularly for such diseases as cancer and acquired immunodeficiency syndrome (AIDS) which are fatal and have no established therapeutic method.

Among others, AIDS is a disease taking the form of immunodeficiency resulting from infect ion of HIV, then in various opportunistic infection diseases, and eventually leading to death without fail. As therapeutic drugs against AIDS currently available include nucleoside reverse transcriptase inhibitors such as azidothymidine (AZT), didanosine (ddI) and sarcitabine (ddC). These drugs have however no function of eliminating already infected cells, and have furthermore problems of a serious side effect and invalidity of drug which may be caused by the appearance of a resistant virus. There is therefore an increasing demand for development of a novel therapeutic drug. As a candidate, the general attention is now attracted by genetic macromolecular sequence such as DNA and RNA which antagonistically exerts effect on multiplication and/or genetic expression of HIV (hereinafter sometimes referred to as "anti-HIV sequence"), including, for example, an antisense strand, ribozyme and decoy relative to a specific genetic region of HIV genom. An antisense is a DNA or an RNA molecule having a sequence complementary to the genomic sequence and is considered to hinder multiplication of virus and inhibit genetic expression by partially forming a double strand with a gene resulting from the virus within a cell. Watsukura, et al. have reported the possibility of inhibiting multiplication of HIV in a cultured cell by adding a chemically synthesized antisense DNA to the culture medium (Proc. Natl. Acad. Sci. U.S.A., 86, 4244-4248, 1989). Ribozyme is an RNA molecule having an activity as that of enzyme of cleaving RNA. Among others, the hammer-head type ribozyme and the hair-pin type ribozyme capable of changing the portion complementarily combining with a substrate into an arbitrary sequence are expected to provide a usefulness as antiviral agents (J. Rossi, et al., Pharmac. Ther. 50, 245-254, 1991: M. Yu, et al., Proc. Natl. Acad. Sci. U.S.A., 90, 6340-6344, 1993). TAR decoy is based on an idea of competitively inhibiting conjuugation of the transacting factor and the cis-acting element by means of competitive molecule. That is, conjugation of HIV's express ion product Tat with mRNa's TAR sequence is considered essential for genetic expression. If there is present an RNA molecule having this TAR sequence (TAR decoy) in a cell, Tat is consumed for the combination with this TAR decoy, making it impossible to combine with mRNA, and hence making it impossible for HIV to grow.

While the anti-HIV sequence as described above has an advantage of permitting easy preparation by means of a DNA synthesizer, problems are that may improvements in stability, solubility and absorbance are required for practical application for clinical purposes and the cost for synthesis is very high.

There are also available many other fundamental studies on genetic therapy to inhibit multiplication of HIV through introduction into an HIV-infected cell by a method of using a plasmid designed to cause these anti-HIV sequences to express within a cell together with calcium phosphate or a cationic lipid, a method based on microinjection, or a method using MoMLV vector (G. Sczakiel, et al., Prospects for Antisense Nucleic Acid Therapy of Cancer and AIDS, 179-193, 1991; Sullenger, B.A., et al., 63, 601-608, 1990; N. Sarver, et al., Science, 247, 1222-1225, 1990; A. Rhodes and W. James, J. General Virology, 71, 1965-1974, 1990). These reports evidence that the anti-HIV sequences express in the cell, and the expression of these sequences successfully inhibits multiplication of HIV having infected the cell. With this gene introducing method using a plasmid vector as described above, however, not only it is difficult to introduce specifically a gene into a target cell in vivo, but also it poses problems including a strong toxicity to cells, complicated gene introducing operation, and a transient expression of the antiviral gene resulting from almost non-occurrence of incorporation of the introduced gene. It is therefore impossible to directly apply this method for clinical purposes.

For these reasons as described above, a genetic therapeutic method is proposed, which uses HIV itself as a vector, as means most suitable for introducing the anti-HIV sequence into an HIV-infected human cell. The use of the HIV vector makes it possible to introduce the anti-HIV sequence into an HIV-infected CD4 positive T cell as a specific target cell.

Incidentally, in order to ensure full display of the effect of the anti-HIV sequence, complementary linkage with an HIV gene. However, because of an insufficient reading of reverse transcriptase, the HIV genome tends to produce a variation for each duplication, and the anti-HIV sequence previously designed to complementarily combine cannot actually combine with the target gene in many cases. It is therefore important to design an anti-HIV sequence with a portion hardly causing such a variation as the target. The tat portion of HIV genome, being relatively hard to cause a variation and because Tat, an expression product thereof, is a control factor required for duplication of HIV, is generally believed to be the most suitable as a target for the anti-HIV sequence such as antisense and ribozyme. For the same reason, the TAR-arranged portion with which Tat is combined is expected to give effect as the affected portion of decoy.

However, the HIV vectors proposed to date have the same LTR sequences at the both ends of genome as those of the wild type. In order for this LTR sequence to function as a promoter of the gene to be introduced (anti-HIV sequence), Tat should have an activity for it. A problem here is therefore that, upon preparing a recombinant human immunodeficiency virus vector, inhibition of expression of the tat portion by an anti-HIV sequence, or sequestration of Tat, the expression product thereof, would seriously impair the titer of the virus vector through deactivation of LTR.

### DISCLOSURE OF INVENTION

The present invention was developed to solve the problems in the conventional HIV vectors, and has an object to provide a novel recombinant HIV vector which ensures expression of an anti-HIV sequence such as antisense, ribozyme or TAR decoy with an HIV-infected cell.

The present invention provides a recombinant HIV vector in which the U3 region of LTR sequence on the genome 5' side is replaced by a promoter sequence of cytomegalovirus.

The present invention provides also a method of manufacturing a recombinant HIV, which comprises transfecting of plasmid vector holding an HIV genome in which the U3 region of LTR sequence at least on the genome 5' side has been replaced by a promoter sequence of cytomegalovirus and a helper plasmid vector holding at least an HIV genome to an animal cell, and extracting HIV particles from a culture medium thereof.

The present invention provides preferable embodiments in which the recombinant HIV vector retains an exotic DNA sequence exerting an antagonistic action on HIV proliferation and/or gene expression, and in which that exotic DNA sequence is a DNA sequence coding an antisense strand to the tat region of the HIV genome, a DNA sequence coding ribozyme to the tat region, or a DNA sequence coding a decoy to the TAR region.

The recombinant HIV vector of the present invention, expressing an anti-HIV sequence such as an antisense, ribozyme or TAR decoy in an HIV-infected cell, is useful as a material for genetic therapy in vivo against an HIV-infected disease.

### BRIEF DESCRIPTION OF DRAWINGS

Fig. 1 is a constitutional diagram of the recombinant plasmid CHXN used in the manufacturing method of the present invention;
Fig. 2 illustrates results of northern blotting on RNA expressing in a cell into which the recombinant plasmid used in the manufacturing method of the present invention has been introduced;
Fig. 3 illustrates results of northern blotting on RNA expressing in a cell into which a recombinant plasmid prepared as a comparative example has been introduced; and
Fig. 4 illustrates the number of drug-resistant colonies for virus particles available by the manufacturing method of the present invention and for the comparative example, respectively.

### BEST MODE FOR CARRYING OUT THE INVENTION

The recombinant HIV vector of the present invention comprises a hybrid-type LTR in which the U3 region of the LTR sequence on the genome 5' side is replaced with a promoter sequence of cytomegalovirus (hereinafter abbreviated as "CMV") (this hybrid-type LTR being hereinafter abbreviated as "CMV-HIV.LTR).

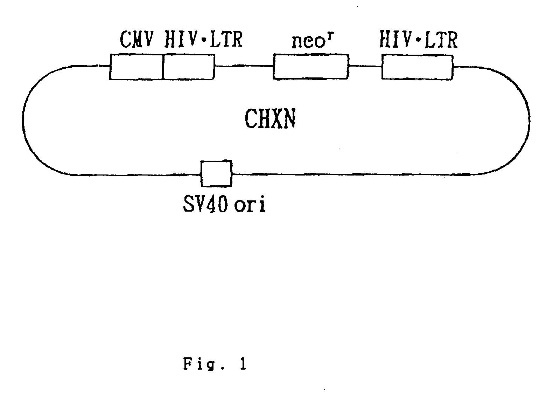

Such a recombinant virus can be manufactured by, for example, the following procedure. The target recombinant HIV vector is available by forming a recombinant plasmid vector retaining a CMV-HIV.LTR at least on the 5' side (for example, see Fig. 1), and a recombinant plasmid vector retaining at least an HIV genome (helper plasmid) individually by the known genetic manipulation, transfecting them into an animal cell by a known method (for example, the calcium phosphate method), culturing this transfectant by a known method, and collecting virus particles in the cultured supernatant. When preparing a recombinant retaining an anti-HIV sequence such as an antisense strand, ribozyme or a decoy, it suffices to insert and connect a DNA sequence coding an anti-HIV sequence to a plasmid vector retaining a CMV-HIV.LTR as shown, for example, in Fig. 1. The anti-HIV sequence thus inserted in retained by the above-mentioned virus particles, stably expresses the antisense strand, ribozyme or the TAR decoy, and thus effectively inhibits multiplication or gene expression of HIV.

### EXAMPLES

The present invention is now described further in detail by means of examples. It should however be noted that the present invention is not limited by these examples.

### Example 1

### (Formation of plasmid)

Operations for forming a plasmid was performed using an ordinary genetic manipulation in all cases. The recombinant plasmid CHXN comprises, sequentially from 5' toward 3' as shown in Fig. 1, CMV-HIV.LTR, neomycin resistant gene, and HIV.LTR, and was formed by inserting these into a plasmid vector including a replication origin of SV40.

### Example 2

### (Transfection of cell by recombinant plasmid, and evaluation of transfected cell)

Transfection was carried out by the usual calcium phosphate method. CHXN obtained in Example 1 in an amount of 10 µg was mixed with a plasmid having Tat code sequence (TAT/LD or CGPE) or a plasmid not retaining Tat code sequence (LH/B), sterilized purified water and aqueous solution of calcium chloride were added into a total amount of 0.5 ml. This mixed solution was dropped while shaking into an HBSP buffer solution in an amount of 0.5 ml, and held at the room temperature for 30 minutes to obtain plasmid-calcium phosphate coprecipitate. As a comparative example, a coprecipitate was prepared in the same manner as in Example 1 also from a plasmid HXN having, sequentially from 5' toward 3', HIV-LTR, a promoter of thymidine kinase resulting from herpesvirus, neomycin-resistant gene, and HIV-LTR, and formed by inserting them into a plasmid having a replication origin of SV40. The individual coprecipitates were added into a culture medium of Cos cell cultured into an about 70% confluent state on a dish having a diameter of 9 cm, and after incubation for four hours in a CO₂ incubator, replaced with a fresh culture medium to further carry out incubation for two days.

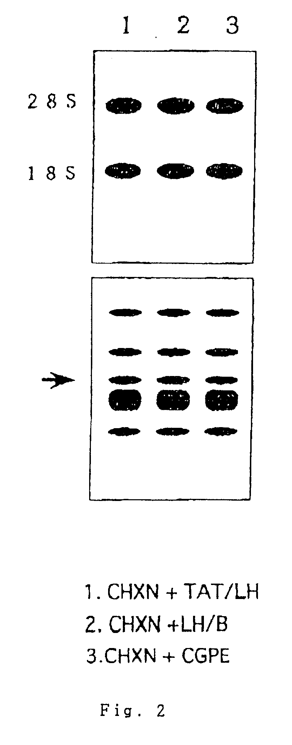

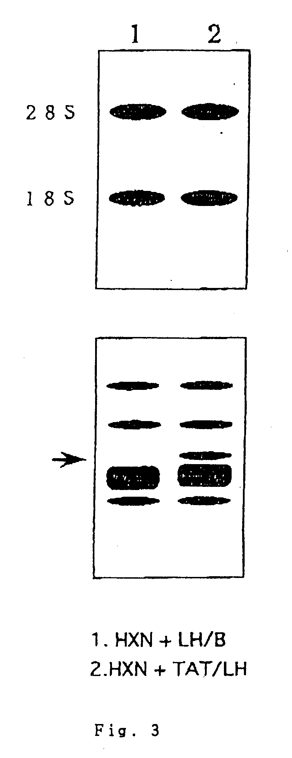

Expression property of the gene introduced into the cells through transfection as described above was evaluated by extracting RNA of these cells by means of cesium chloride by the density gradient centrifugation, and analyzing by the northern blotting method. The results are shown in Figs. 2 and 3.

While expression of RNA is observed in a manner dependent upon Tat in the comparative example (plasmid HXN) (Fig. 3), expression of RNA is observed in CHXN, irrespective of the presence of Tat (Fig. 2).

### Example 3

### (Preparation of recombinant virus and transduction of cells)

CHXN or HXN was transfected, together with CCPE, in the same manner as in Example 2. After the lapse of two days from the transfection, a culture supernatant was collected to use as a virus solution. On the day before collection of the virus solution, 2 x 10⁵ CD4 positive Hela cells had been seeded per 65-mm dish, and culture supernatant of cells not subjected to transfection was added in an amount of 0.5 ml or 3 ml, together with 30 µg polyprene (transduction) as these virus solutions or control groups thereof. After holding the product in a CO₂ incubator for two days, cells were stripped off the dish by means of a trypsin-EDTA mixed solution, and seeding was conducted again onto a 85-mm dish. After incubation for six hours in a CO₂ incubator, G-41 was added into the culture medium in an amount of 750 µg/ml. After another incubation for seven days, the number of colonies having acquired drug resistance was counted.

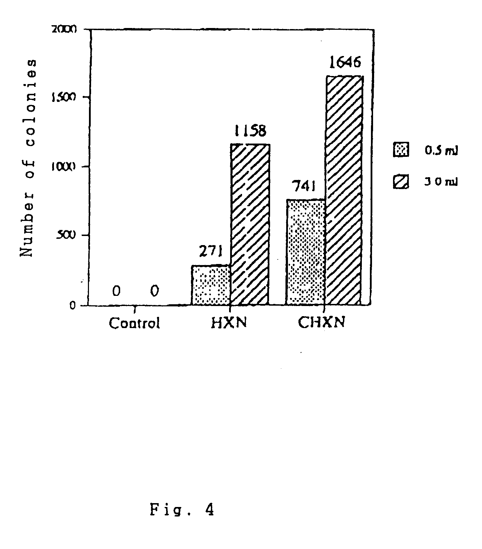

The results are shown in Fig. 4. No colony was observed in the control groups not having received gene introduction by virus. In the CHXN groups, on the other hand, a number of colonies equal to, or even superior to that in the HXN groups was observed in the CHXN groups. These results suggest that a recombinant virus can be prepared transfection of CHXN or CCPE, that this virus has the ability to introduce genes into target cells, and that the recombinant genes introduced into cells efficiently achieve expression.

### INDUSTRIAL APPLICABILITY

The virus vector of the present invention is applicable to genetic therapy in vivo against HIV-infected diseases.

## Claims

1. A recombinant human immunodeficiency virus vector wherein the U3 region of an LTR sequence on the genome 5' side is replaced by a promoter sequence of cytomegalovirus.

2. A recombinant human immunodeficiency virus vector as claimed in claim 1, wherein said vector retains an exotic DNA sequence which antagonistically exerts an action on multiplication and/or genetic expression of the human immunodeficiency virus.

3. A recombinant human immunodeficiency virus vector as claimed in claim 2, wherein said exotic DNA sequence is a DNA sequence which codes an antisense to the tat region of the human immunodeficiency virus genome.

4. A recombinant human immunodeficiency virus vector as claimed in claim 2, wherein said exotic DNA sequence is a DNA sequence which codes a ribozyme to the tat region of the human immunodeficiency virus genome.

5. A recombinant human immunodeficiency virus vector as claimed in claim 2, wherein said exotic DNA sequence is a DNA sequence which codes a decoy of the TAR sequence of the human immunodeficiency virus genome.

6. A method of manufacturing a recombinant human immunodeficiency virus, which comprises the steps of transfecting a plasmid vector retaining a human immunodeficiency virus genome in which at least the U3 region of an LTR sequence on the genome 5' side is replaced by a promoter sequence of cytomegalovirus and a helper plasmid vector retaining at least human immunodeficiency virus genome into animal cells, and collecting human immunodeficiency virus particles from a culture medium thereof.

7. A method of manufacturing a human immunodeficiency virus as claimed in claim 6, wherein said plasmid vector retaining the human immunodeficiency virus genome in which the U3 region of the LTR sequence on the genome 5' side is replaced by a promoter sequence of cytomegalovirus retains also an exotic DNA sequence which antagonistically exerts an action on multiplication and/or gene expression of human immunodeficiency virus.

8. A method of manufacturing a human immunodeficiency virus as claimed in claim 7, wherein said exotic DNA sequence is a DNA sequence which codes a decoy to an antisense strand, ribozyme or TAR sequence to the tat region of the human immunodeficiency virus genome.
